(19) Europäisches Patentamt
European Patent Office
Office européen des brevets

(11) EP 4 344 644 A1

(12) EUROPEAN PATENT APPLICATION

(43) Date of publication:
03.04.2024 Bulletin 2024/14

(21) Application number: 22198710.0

(22) Date of filing: 29.09.2022

(51) International Patent Classification (IPC):
A61B 6/02 (2006.01)          A61B 6/00 (2024.01)
G06T 7/00 (2017.01)          G06T 11/00 (2006.01)

(52) Cooperative Patent Classification (CPC):
A61B 6/502; A61B 6/025; A61B 6/463;
A61B 6/5211; G06T 11/005; G06T 2211/436;
G06T 2211/441

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Siemens Healthineers AG
91301 Forchheim (DE)

(72) Inventors:
• Eckert, Dominik
90762 Fürth (DE)

• Kappler, Steffen
91090 Effeltrich (DE)
• Ritschl, Ludwig
96155 Buttenheim (DE)

(74) Representative: Siemens Healthineers
Patent Attorneys
Siemens Healthcare GmbH
SHS TE IP
Henkestraße 127
91052 Erlangen (DE)

(54) **COMPUTER-IMPLEMENTED METHOD FOR PROVIDING A FIRST X-RAY IMAGE AND A SECOND X-RAY IMAGE ACQUIRED BY AN X-RAY IMAGING SYSTEM**

(57) The invention relates to a computer-implemented method (10) for providing a first X-ray image and a second X-ray image acquired by an X-ray imaging system, comprising:
- Receiving (12) an X-ray image dataset of an examination region,
- Applying (13) a first image impression function to the X-ray image dataset and generating a first X-ray image with a first image impression,
- Applying (14) a second image impression function to the X-ray image dataset and generating a second X-ray image with a second image impression,
- Providing (15) the first X-ray image and the second X-ray image.

FIG 1

EP 4 344 644 A1

**Description**

The invention relates to a computer-implemented method for providing a first X-ray image and a second X-ray image acquired by an X-ray imaging system, an X-ray imaging system, a computer program product, and a computer-readable medium, wherein an X-ray image can be provided with different image impressions to the user.

An X-ray imaging system, e.g. a radiography system or a fluoroscopy system, comprises an X-ray source and an X-ray detector. The examination object, in particular a patient, is arranged between the X-ray source and the X-ray detector so that an X-ray image of an examination region can be acquired.

A mammography system includes an X-ray source and an X-ray detector, with a breast arranged between the X-ray source and the X-ray detector. The examination area comprises the breast, especially the entire breast. In general, the breast is compressed or fixed by means of a compression unit. For this purpose, in particular, the surface of the X-ray detector or a housing of the X-ray detector and an essentially parallel arranged compression plate can be used. Furthermore, other forms of the compression unit are known. An X-ray data set can be generated. The X-ray data set may include a digital full-field mammography image and / or a tomosynthesis image.

Modern mammography systems offer a tomosynthesis function to generate a series of projections that allow a three-dimensional representation of the breast using reconstructed layers. Here, the X-ray emitter or the X-ray source moves in an angular range of 15 to 50 degrees over the compressed breast. The digital full-field mammography image and the tomosynthesis image can also be acquired together within a single compression (per breast).

X-rays images can have different image impressions called flavors. These flavors depend on the hardware used to record the x-ray images as well as on the operation principle and the parameter set of the applied reconstruction and post processing algorithm. The influence of the chosen recording system on the x-ray image impression can be observed in radiography as well as in mammography. For example, two reconstructed x-ray images of the same breast but recorded with a different parameter set of the post processing algorithm can exhibit different image impressions.

Radiologists are used to a particular flavor or image impression. Changing or working with different image impression, e.g. depending on the body region, leads to a decrease in the reading time and the reliability of the diagnosis. This is especially problematic, if different x-ray images with different flavors must be compared against each other. However, there are several reasons, which make the use or exchange of various x-ray systems necessary: An x-ray system exchange or update with more recent technology increases the image quality and consequently the reliability of the diagnosis. An old x-ray machine must be replaced due to a defect, or a radiologist changes the working side.

When a new system or software version is installed at a customer site, service specialists or image quality specialists can adjust post-processing parameters by hand together with the customer, using some example cases from the local PACS. This is very time consuming, and the results are not always satisfying. Especially for breast imaging, the style adjustment depends on the content of the example cases used for the adjustment process. Characteristics like the density of the breast, thickness or view can lead to different post-processing parameter sets in the end. To overcome this problem, several cases are used for this process, still leading to non-ideal results. And the problem is even stronger when adapting the style of another vendors system.

It is an object of the invention to provide a computer-implemented method for providing a first X-ray image and a second X-ray image acquired by an X-ray imaging system, a computer program product, a computer-readable medium, and an X-ray imaging system, which makes it easier for the user to adapt to a new or different image impression.

The object of the invention is solved by a computer-implemented method for providing a first X-ray image and a second X-ray image acquired by an X-ray imaging system according to claim 1, an X-ray imaging system according to claim 12, a computer program product according to claim 14, and a computer-readable medium according to claim 15.

In the following the solution according to the invention is described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the system.

Furthermore, in the following the solution according to the invention is described with respect to methods and systems providing a first X-ray image and a second X-ray image acquired by an X-ray imaging system as well as with respect to methods and systems for the training of the trained function. Features, advantages or alternative embodiments herein can be assigned to the other claimed objects and vice versa. In other words, claims for methods and systems for training of the trained function can be improved with features described or claimed in context of the methods and systems providing a first X-ray image and a second X-ray image acquired by an X-ray imaging system, and vice versa.

In particular, the trained function of the methods and systems providing a first X-ray image and a second X-ray image acquired by an X-ray imaging system can be adapted by the methods and systems for training of the trained function. Furthermore, the input data can comprise advantageous features and embodiments of the training input data, and vice versa. Furthermore, the output data can comprise advantageous features and embodiments of the output training data, and vice versa.

The invention relates to a computer-implemented method for providing a first X-ray image and a second X-ray image acquired by an X-ray imaging system., comprising:

- Receiving an X-ray image dataset of an examination region,
- Applying a first image impression function to the X-ray image dataset and generating a first X-ray image with a first image impression,
- Applying a second image impression function to the X-ray image dataset and generating a second X-ray image with a second image impression,
- Providing the first X-ray image and the second X-ray image.

The first and second image impression function can be a set of parameters which is applied to the X-ray image dataset. The first and second image impression function can be a trained function which is applied to the X-ray image dataset. The image impression can be defined by parameters like noise, contrast, artefact reduction algorithms and others.

According to an aspect of the invention, the first X-ray image and the second X-ray image are displayed in a toggle mode.

According to an aspect of the invention, the first X-ray image and the second X-ray image are displayed concurrently.

According to an aspect of the invention, applying a second image impression comprises applying a trained algorithm to the X-ray image dataset.

According to an aspect of the invention, the trained algorithm is based on a deep learning method.

According to an aspect of the invention, the trained algorithm is based on a non-linear algorithm.

According to an aspect of the invention, the second image impression mimics the use of another X-ray imaging system.

According to an aspect of the invention, the second image impression mimics the use of other acquisition parameters for acquiring the X-ray image dataset.

According to an aspect of the invention, the second image impression mimics the use of other post-processing methods to the X-ray image dataset.

According to an aspect of the invention, the first X-ray image is appropriate for diagnostic evaluation.

According to an aspect of the invention, the first X-ray image and the second X-ray image comprise the same diagnostic relevant information.

The invention further relates to an X-ray imaging system for carrying out the method according to the invention.

According to an aspect of the invention, the X-ray imaging system is a radiography system or a mammography system.

The invention further relates to a computer program product comprising instructions which, when the program is executed by the X-ray imaging system according to the invention, cause the X-ray system to carry out the method according to the invention.

The invention further relates to a computer-readable medium comprising instructions which, when executed by the X-ray imaging system according to the invention, cause the X-ray imaging system to carry out the method according to the invention.

The invention further relates to a computer-implemented method for providing a trained function, comprising:

- receiving input training data, wherein the input training data comprises a first X-ray image with a first image impression,
- receiving output training data, wherein the output training data is related to the input training data, wherein the output training data comprises a second X-ray image with a second image impression,
- training a function based on the input training data and the output training data,
- providing the trained function.

The invention further relates to a training system, comprising:

- A first training interface, configured for receiving input training data, wherein the input training data comprises a first X-ray image with a first image impression,
- A second training interface, configured for receiving output training data, wherein the output training data is related to the input training data, wherein the output training data comprises a second X-ray image with a second image impression,
- A training computation unit, configured for training a function based on the input training data and the output training data,
- A third training interface, configured for providing the trained function.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

To help the radiologist to adapt to a new flavor or a different image impression, the inventors propose the use of an additional x-ray image besides the usual image which is used for diagnosis. The second x-ray image is basing on the same recording than the default image but is post-processed to have another flavor or image impression. The radiologist can be able to alternate or to toggle between the standard x-ray image and the additional one. The additional (second) image allows the radiologist to see flavor versions or different image impressions, which were used on previous systems or which he was used to. This is especially helpful, if several x-ray images with different flavors must be compared to each other, because the new x-ray images can be then also shown in another image impression, so that all images to be compared exhibit the same image impression. Furthermore, the additional flavor or additional image impression helps the radiologist during the transition to a new system and new image impression, since he is now able to switch back to his old version. He can then compare how different areas of the X-ray image are represented by the new image impression.

Because of the existence of the first X-ray image, the second X-ray image must not be certified to be used for diagnosis. Hence it must not be proven that diagnostically relevant information is not altered. This allows more degrees of freedom in the development of the post processing chain for the second x-ray image.

Machine learning algorithms for a style/flavor transfer or the transfer of image impressions can be used in particular. X-ray images with the image impression requested by the radiologist can serve as objective data to fit the parameter of the machine learning algorithm. The image impression of x-ray images from the new system can be then transformed to meet the image impression of the objective data by the algorithm. This transformation can take place on the post processed images, on raw images or unfinished processed x-ray images.

The idea of an additional second X-ray image, which serves the radiologist as a reference image comprising a known image impression is helpful during the phase of moving to a new X-ray imaging system. The additional second image allows the radiologist to see image impression versions which were used on previous systems or which he was used to. This is especially helpful, if several x-ray images with different image impressions must be compared to each other because the new x-ray images can be also shown in another image impression, so that all X-ray images which must be compared have the same image impression. Furthermore, the additional image impression helps the radiologist by the transition to a new system and new image impression, since he is now able to switch back to his old version. He can then compare how different areas of the image are represented by the new image impression. Because of the existence of the first image, the second x-ray image must not be certified to be used for diagnosis. This additional image allows the use of machine learning algorithm to perform a style transfer or a transfer of image impression.

Examples of embodiments of the invention are explained in more detail below by means of drawings.

FIG 1 a schematic representation of the method for providing a first X-ray image and a second X-ray image acquired by an X-ray imaging system;

FIG 2 a schematic representation of a neural network according to the invention; and

FIG 3 a schematic representation of a convolutional neural network according to the invention.

FIG 4 a schematic representation of a mammography system; and

FIG 5 a schematic representation of an X-ray imaging system.

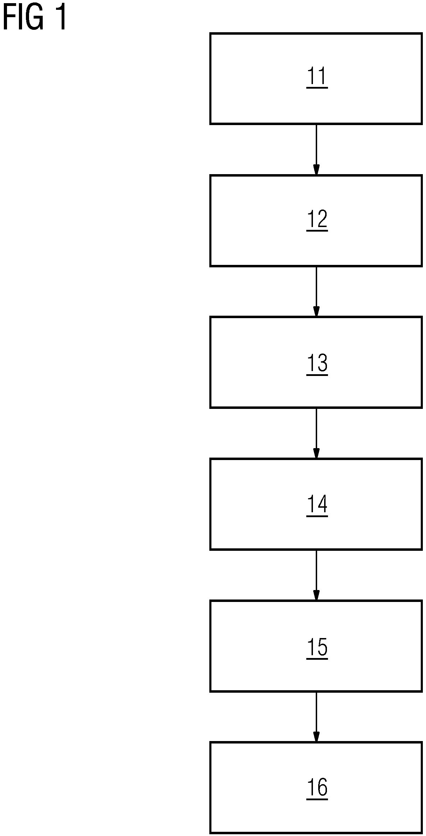

Fig. 1 displays a computer-implemented method 10 for providing a first X-ray image and a second X-ray image acquired by an X-ray imaging system according to the invention. The method 10 comprises the following steps, preferably in the following order:

- Receiving 12 an X-ray image dataset of an examination region,
- Applying 13 a first image impression function to the X-ray image dataset and generating a first X-ray image with a first image impression,

- Applying 14 a second image impression function to the X-ray image dataset and generating a second X-ray image with a second image impression,
- Providing 15 the first X-ray image and the second X-ray image.

Before receiving 12 the X-ray image dataset, the X-ray image dataset can be acquired in a step 11. After providing 15 the first X-ray image and the second X-ray image, the first X-ray image and the second X-ray image can be displayed in step 16.

The first X-ray image and the second X-ray image are displayed in a toggle mode. In an alternative embodiment, the first X-ray image and the second X-ray image are displayed concurrently.

Applying a second image impression can comprise applying a trained algorithm to the X-ray image dataset, wherein the trained algorithm is based on a deep learning method or wherein the trained algorithm is based on a non-linear algorithm.

The second image impression can mimic the use of another X-ray imaging system, the use of other acquisition parameters for acquiring the X-ray image dataset, and/or the use of other post-processing methods to the X-ray image dataset. The first X-ray image is appropriate for diagnostic evaluation. The first X-ray image and the second X-ray image comprise the same diagnostic relevant information.

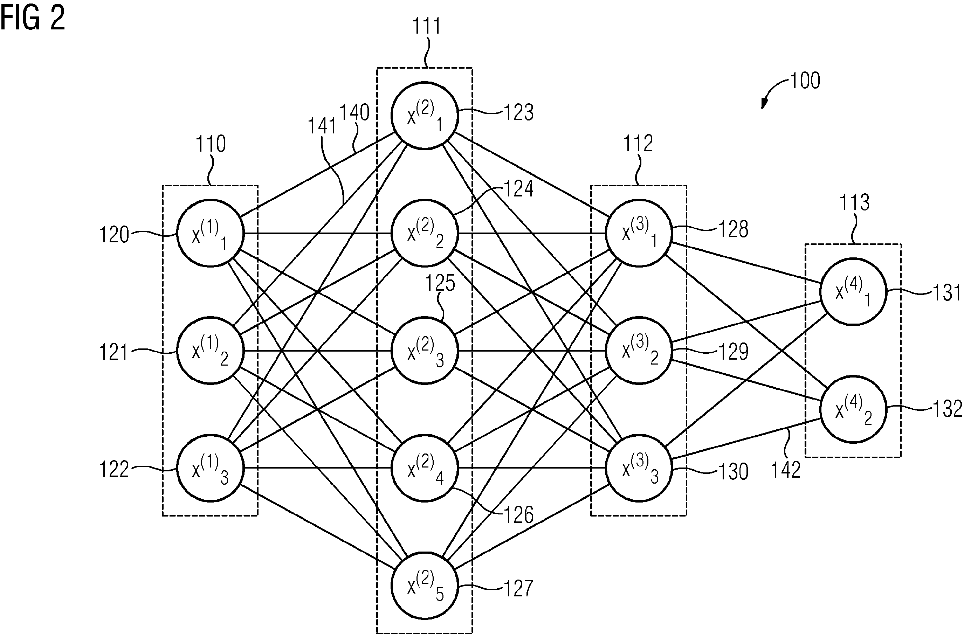

Fig. 2 displays an embodiment of an artificial neural network 100. Alternative terms for "artificial neural network" are "neural network", "artificial neural net" or "neural net".

The artificial neural network 100 comprises nodes 120, ..., 132 and edges 140, ..., 142, wherein each edge 140, ..., 142 is a directed connection from a first node 120, ..., 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in Fig. 1 the edge 140 is a directed connection from the node 120 to the node 123, and the edge 142 is a directed connection from the node 130 to the node 132. An edge 140, ..., 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the artificial neural network 100 can be arranged in layers 110, ..., 113, wherein the layers can comprise an intrinsic order introduced by the edges 140, ..., 142 between the nodes 120, ..., 132. In particular, edges 140, ..., 142 can exist only between neighboring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network.

In particular, a (real) number can be assigned as a value to every node 120, ..., 132 of the neural network 100. Here, $x^{(n)}_i$ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, $w^{(m,n)}_{i,j}$ denotes the weight of the edge between the i-th node 120, ..., 132 of the m-th layer 110, ..., 113 and the j-th node 120, ..., 132 of the n-th layer 110, ..., 113. Furthermore, the abbreviation $w^{(n)}_{i,j}$ is defined for the weight $w^{(n,n+1)}_{i,j}$.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by

$$x_j^{(n+1)} \; = \; f\left( \sum_i x_i^{(n)} \cdot w_{i,j}^{(n)} \right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values $w^{(m,n)}_{i,j}$ for the edges, the neural network 100 has to be trained using training data.

In particular, training data comprises training input data and training output data (denoted as $t_i$). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left( \sum_k \delta^{(n+1)}_k \cdot w^{(n+1)}_{j,k} \right) \cdot f' \left( \sum_i x^{(n)}_i \cdot w^{(n)}_{i,j} \right)$$

based on $\delta^{(n+1)}_j$, if the (n+1)-th layer is not the output layer, and

$$\delta^{(n)}_j = \left( x^{(n+1)}_k - t^{(n+1)}_j \right) \cdot f' \left( \sum_i x^{(n)}_i \cdot w^{(n)}_{i,j} \right)$$

if the (n+1)-th layer is the output layer 113, wherein f' is the first derivative of the activation function, and $y^{(n+1)}_j$ is the comparison training value for the j-th node of the output layer 113.

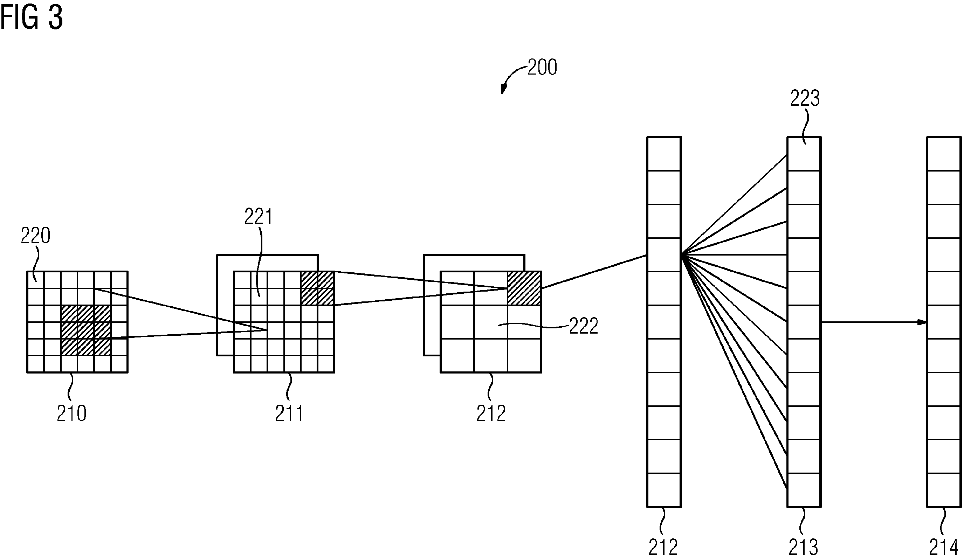

Fig. 3 displays an embodiment of a convolutional neural network 200. In the displayed embodiment, the convolutional neural network comprises 200 an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as $x^{(n)}$ [i,j]. However, the arrangement of the nodes 220, ..., 224 of one layer 210, ..., 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values $x^{(n)}_k$ of the nodes 221 of the convolutional layer 211 are calculated as a convolution $x^{(n)}_k = K_k * x^{(n-1)}$ based on the values $x^{(n-1)}$ of the nodes 220 of the preceding layer 210, where the convolution * is defined in the two-dimensional case as

$$x^{(n)}_k[i,j] = (K_k * x^{(n-1)})[i,j] = \sum_{i'} \sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a non-linear pooling function f. For example, in the two dimensional case the values $x^{(n)}$ of the nodes 222 of the pooling layer 212 can be calculated based on the values $x^{(n-1)}$ of the nodes 221 of the preceding layer 211 as

$$x^{(n)}[i,j] = f(x^{(n-1)}[id_1, jd_2], ..., x^{(n-1)}[id_1+d_1-1, jd_2+d_2-1])$$

In other words, by using a pooling layer 212 the number of nodes 221, 222 can be reduced, by replacing a number $d_1 \cdot d_2$ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced.

This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = max(0,x)$, the tangent hyperbolics function or the sigmoid function.

In particular, convolutional neural networks 200 can be trained based on the backpropagation algorithm. For preventing overfitting, methods of regularization can be used, e.g. dropout of nodes 220, ..., 224, stochastic pooling, use of artificial data, weight decay based on the L1 or the L2 norm, or max norm constraints.

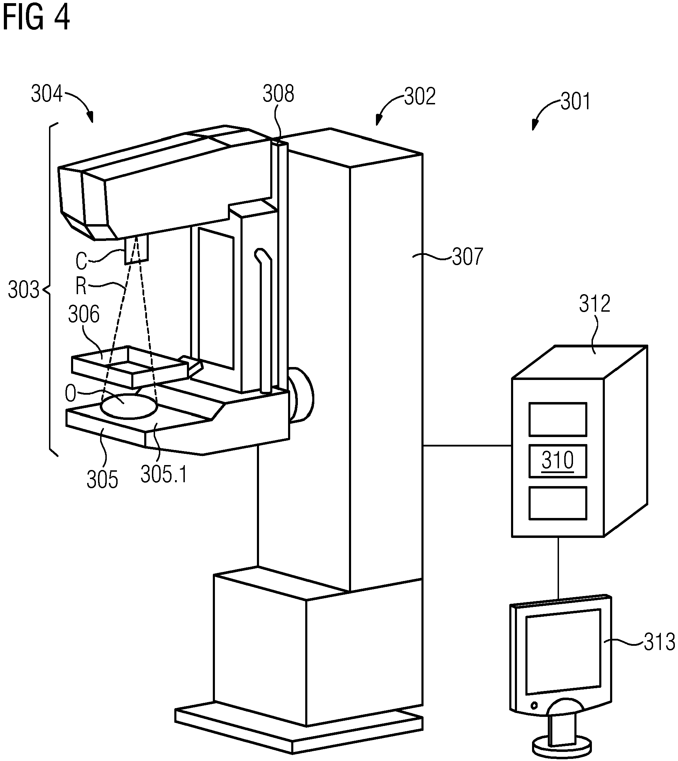

In Figure 4, a mammography system 301, in particular in the form of a tomosynthesis system, is shown by way of example and roughly schematically. Relative directions such as "above", "below", etc. refer to a tomosynthesis system set up for operation as intended. The mammography system 301 includes a tomosynthesis device 302 and a control device 312. The tomosynthesis device 302 has a standing column 307 and a source-detector arrangement 303, which in turn comprise an X-ray source 304 and an X-ray detector 305 with a detector area 5.1. Standing column 307 is in operation on the ground. The source-detector arrangement 303 can be connected to it in a movable manner, so that the height of the detector surface 305.1, i.e. the distance to the substrate, can be adjusted to a chest height of a patient.

A breast O of the patient (shown schematically here) is the object of examination, the breast O, for an examination on the top of the detector surface 305.1. Above the chest O and the detector surface 305.1 a compression plate 306 is arranged, which is movably connected to the source-detector arrangement 303. For the examination, the breast O is compressed and at the same time fixed by lowering the compression plate 306 to it, so that pressure is applied on the breast O between compression plate 306 and detector surface 305.1.

The X-ray emitter 304 is arranged and designed opposite the X-ray detector 305 in such a way that the X-ray detector 305 detects X-rays R emitted by it after at least part of the X-ray radiation R has penetrated the patient's breast O. The X-ray emitter 304 is relative to the X-ray detector 305 by means of a rotary arm 308, for example, in a range of ± 25° around a basic position, in which it is perpendicular to the detector surface 305.1.

The mammography system 301 can in particular a control device 12 and a computer unit with a training unit 310. The control device 312 is connected to a terminal 313, for example having a user interface or display unit, through which a user can communicate commands to the tomosynthesis system 301 or retrieve measurement results, for example the X-ray. The control device 312 may be located in the same room as the tomosynthesis device 302, but it may also be located in an adjacent control room or at an even greater spatial distance.

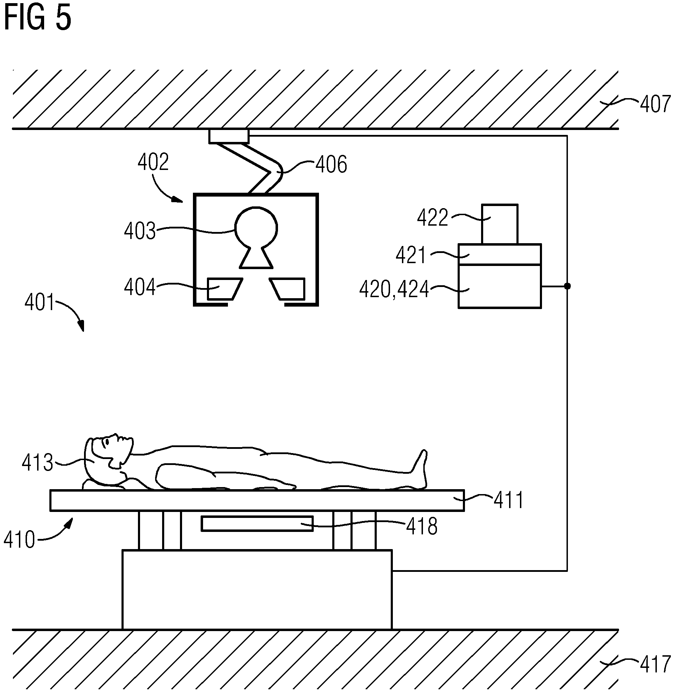

Fig. 5 shows an exemplary embodiment of an X-ray (imaging) system 401, especially a radiography system, according to the invention. The X-ray system 401 has a patient positioning device 410 with a table 411 fixed to the floor 417. The object 413 lies on the table 411. The patient positioning device 410 further comprises an X-ray detector unit 418.

The X-ray system 401 comprises an X-ray source 403 and an X-ray detector unit 418. The X-ray source unit 402, which comprises the X-ray source 403 and a collimator unit 404. The X-ray source unit 402 can be connected to the ceiling 407 of the examination room by means of a ceiling mount 406. By means of the ceiling mount 406, the X-ray source 403 can be moved.

The X-ray system 401 may also comprise an input unit 421 and an output unit 422. The input unit 421 and the output unit 422 may be connected to the control unit 420. The control unit 420 may further comprise or be connected to the training unit 424.

Although the invention has been further illustrated in detail by the preferred embodiments, the invention is not limited by the disclosed examples and other variations may be derived therefrom by those skilled in the art without departing from the scope of protection of the invention.

**Claims**

1. A computer-implemented method (10) for providing a first X-ray image and a second X-ray image acquired by an X-ray imaging system, comprising:

   - Receiving (12) an X-ray image dataset of an examination region,
   - Applying (13) a first image impression function to the X-ray image dataset and generating a first X-ray image with a first image impression,
   - Applying (14) a second image impression function to the X-ray image dataset and generating a second X-ray image with a second image impression,
   - Providing (15) the first X-ray image and the second X-ray image.

2. A method according to claim 1, wherein the first X-ray image and the second X-ray image are displayed in a toggle mode.

3. A method according to claim 1, wherein the first X-ray image and the second X-ray image are displayed concurrently.

4. A method according to the preceding claims, wherein applying a second image impression comprises applying a trained algorithm to the X-ray image dataset.

5. A method according to claim 4, wherein the trained algorithm is based on a deep learning method.

6. A method according to claim 4, wherein the trained algorithm is based on a non-linear algorithm.

7. A method according to the preceding claims, wherein the second image impression mimics the use of another X-ray imaging system.

8. A method according to the preceding claims, wherein the second image impression mimics the use of other acquisition parameters for acquiring the X-ray image dataset.

9. A method according to the preceding claims, wherein the second image impression mimics the use of other post-processing methods to the X-ray image dataset.

10. A method according to the preceding claims, wherein the first X-ray image is appropriate for diagnostic evaluation.

11. A method according to the preceding claims, wherein the first X-ray image and the second X-ray image comprise the same diagnostic relevant information.

12. An X-ray imaging system (401) for carrying out the method according to the claims 1 to 11.

13. An X-ray imaging system according to claim 12, wherein the X-ray imaging system is a radiography system or a mammography system (301).

14. A computer program product comprising instructions which, when the program is executed by the X-ray imaging system according to one of the claims 12 to 13, cause the X-ray system to carry out the method according to one of the claims 1 to 11.

15. A computer-readable medium comprising instructions which, when executed by the X-ray imaging system according to one of the claims 12 to 13, cause the X-ray imaging system to carry out the method according to one of the claims 1 to 11.

FIG 1

FIG 2

EP 4 344 644 A1

FIG 3

FIG 4

# FIG 5

Europäisches
Patentamt

European
Patent Office

Office européen
des brevets

# EUROPEAN SEARCH REPORT

Application Number

EP 22 19 8710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 722 003 A1 (DENTAL IMAGING TECHNOLOGIES CORP [US]) 23 April 2014 (2014-04-23) * paragraph [0021] – paragraph [0060]; figures * ----- | 1,2,7, 10-15 | INV. A61B6/02 A61B6/00 G06T7/00 G06T11/00 |
| X | US 2013/051527 A1 (SAKAGUCHI TAKUYA [JP] ET AL) 28 February 2013 (2013-02-28) * paragraph [0022] – paragraph [0065]; figures * ----- | 1-3,8, 10-15 | |
| X | US 2022/092768 A1 (MELAPUDI VIKRAM [IN] ET AL) 24 March 2022 (2022-03-24) * paragraph [0022] – paragraph [0071]; figures * ----- | 1,3-5, 10-15 | |
| X | US 2022/096033 A1 (BEISTER MARCEL [DE] ET AL) 31 March 2022 (2022-03-31) * paragraph [0125] – paragraph [0162]; figures * ----- | 1,3-6, 9-15 | |
| X | US 2020/163638 A1 (WICKLEIN JULIA [DE] ET AL) 28 May 2020 (2020-05-28) * paragraph [0065] – paragraph [0096]; figures * ----- | 1,3,8-15 | TECHNICAL FIELDS SEARCHED (IPC) A61B G06T |

The present search report has been drawn up for all claims

| Place of search | Date of completion of the search | Examiner |
|---|---|---|
| Munich | 23 February 2023 | Strubel, Christine |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................
& : member of the same patent family, corresponding
    document

EPO FORM 1503 03.82 (P04C01)

2

## ANNEX TO THE EUROPEAN SEARCH REPORT
## ON EUROPEAN PATENT APPLICATION NO.

EP 22 19 8710

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2023

| Patent document cited in search report | | Publication date | Patent family member(s) | | Publication date |
|---|---|---|---|---|---|
| EP 2722003 | A1 | 23-04-2014 | BR 102013019944 | A2 | 10-11-2015 |
| | | | CN 103777913 | A | 07-05-2014 |
| | | | EP 2722003 | A1 | 23-04-2014 |
| | | | JP 5666650 | B2 | 12-02-2015 |
| | | | JP 2014083428 | A | 12-05-2014 |
| | | | KR 20140049919 | A | 28-04-2014 |
| | | | US 2014111535 | A1 | 24-04-2014 |
| US 2013051527 | A1 | 28-02-2013 | CN 102970932 | A | 13-03-2013 |
| | | | JP 2013017511 | A | 31-01-2013 |
| | | | US 2013051527 | A1 | 28-02-2013 |
| | | | WO 2013005805 | A1 | 10-01-2013 |
| US 2022092768 | A1 | 24-03-2022 | NONE | | |
| US 2022096033 | A1 | 31-03-2022 | DE 102020212382 | B3 | 20-01-2022 |
| | | | US 2022096033 | A1 | 31-03-2022 |
| US 2020163638 | A1 | 28-05-2020 | EP 3657442 | A1 | 27-05-2020 |
| | | | US 2020163638 | A1 | 28-05-2020 |

EPO FORM P0459

For more details about this annex : see Official Journal of the European Patent Office, No. 12/82